# EUROPEAN PATENT APPLICATION

(11) **EP 2 522 347 A1**
(43) Date of publication of application: **14.11.2012**
(21) Application number: 12168958.2
(22) Date of filing: 29.06.2006
(51) Int. Cl.: A61K 31/353, A61P 9/14, A61K 31/70, A61K 31/35

(54) **Flavonols and procyanidins for inducing peripheral blood vessel vasodilation**

(30) Priority: 29.06.2005 US 695557 P
(62) Divisional of application: 06785871.2
(71) Applicant: Mars Incorporated, McLean, Virginia 22101-3883 (US)
(72) Inventor: Kwik-Uribe, Catherine L, Stroudsburg, PA Pennsylvania 18360 (US); Schmitz, Harold H, 20814, MD Maryland Bethesda (US); Kelm, Mark A, Fresno, CA California 93722 (US); Hammerstone, John F, Rockville, MD Maryland 20850 (US)
(74) Representative: Keen, Celia Mary

(57) **Abstract**

The invention relates to compositions, and methods of use thereof, containing polyphenols such as flavanols, procyanidins, their derivatives and epimers thereof, for inducing peripheral blood vessel vasodilation, for example for treating or preventing peripheral vascular diseases.

## Description

This application claims the benefit, under 35 USC Section 119, of the U.S. Provisional Application Serial No. 60/695,557 filed June 29, 2005, the disclosure of which is hereby incorporated herein by reference.

### FIELD OF THE INVENTION

The invention relates to compositions, and methods of use thereof, containing polyphenols such as flavanols, procyanidins and derivatives thereof for inducing peripheral blood vessel vasodilation, for example for treating or preventing peripheral vascular diseases.

### BACKGROUND OF THE INVENTION

Vasodilation is the widening of blood vessels resulting from relaxation of the muscular wall of the vessels. Vasodilation can alleviate disease and disorders of the cardiovascular system, for example hypertension. Hypertension is a leading cause of cardiovascular diseases, including stroke, heart attack, heart failure, irregular heart beat and kidney failure. Hypertension is a condition where the pressure of blood within the blood vessels is higher than normal as it circulates through the body. When the systolic pressure exceeds 150 mm Hg or the diastolic pressure exceeds 90 mm Hg for a sustained period of time, damage is done to the body. For example, excessive systolic pressure can rupture blood vessels. When such rupture occurs within the brain, a stroke results. Hypertension can also cause thickening and narrowing of the blood vessels which can lead to atherosclerosis. Elevated blood pressure can also force the heart muscle to enlarge as it works harder to overcome the elevated resting (diastolic) pressure when blood is expelled. This enlargement can eventually produce irregular heartbeats or heart failure.

The regulation of blood pressure is a complex event where one of the known mechanism involves nitric oxide (NO) produced by the constitutive Ca⁺²/calmodulin dependent form of nitric oxide synthase (NOS). NO produces muscle relaxation in the vessel (dilation), which lowers the blood pressure. When the normal level of NO is not produced, either because production is blocked by an inhibitor, or in pathological states, such as atherosclerosis, the vascular muscles do not relax to the appropriate degree. The resulting vasoconstriction may increase regional blood pressure and may be responsible for some forms of hypertension.

Peripheral vascular disease and other conditions affecting peripheral and/or small blood vessels can also benefit from vasodilation. All blood vessels that are surrounded by SMC can dilate in response to changes in NO. However, in general, the large blood vessels respond strongly to NO. As one moves into arterioles, the vessels are more closely linked with tissue beds, these vessels are influenced to dilate not only in response to increased NO production by endothelial cells, but is also in response to regional changes in the levels of other vasodilators, compounds such as adenosine and prostaglandin I2 (these compounds act directly on SMC to induce the NO-independent relaxation). Moreover, when endothelium is damaged or so compromised that NO is not enough to sufficiently relax the vascular system, other vasodilating agents need to be used. Applicants have discovered that compounds described herein can be used for applications that can benefit from NO-independent vasodilation.

### SUMMARY OF THE INVENTION

The invention relates to compositions, products and methods of inducing NO-independent vasodilation and treating/preventing related diseases and conditions.

In one aspect, the invention relates to a composition, such as a pharmaceutical, a food, a food additive, or a dietary supplement comprising the compounds of the invention such as flavanols, procyanidins or their derivatives or epimers thereof. The composition may optionally contain an additional vasodilating therapeutic agent, or may be administered in combination with an additional therapeutic agent. Packaged products containing the above-mentioned compositions and a label and/or instructions for use to improve vasodilation in peripheral and/or small blood vessels and/or to treat/prevent diseases and conditions recited herein are also within the scope of the invention.

In another aspect, the invention relates to methods of treating conditions or diseases associated with peripheral vascular circulation and/or small blood vessels by administering to a mammal, such as a human or a veterinary animal, an effective amount of a flavanol, a procyanidin or a derivative thereof, or an epimer thereof.

### DETAILED DESCRIPTION

All patents, patent applications and references cited in this application are hereby incorporated herein by reference. In case of any inconsistency, the present disclosure governs.

The invention relates to a composition comprising flavanols, procyanidins, their derivatives, or epimers thereof. As used herein, the term "flavanol" refers to a monomer and the term "procyanidin" refers to an oligomer.

The present invention relates to a composition comprising an effective amount of the compound having the following formula Aₙ, or a pharmaceutically a 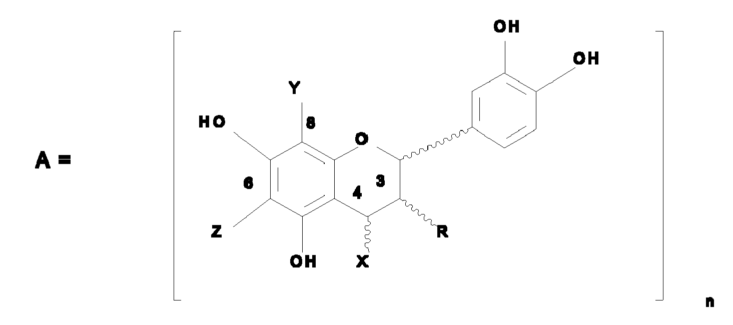 cceptable salt or derivative thereof (including oxidation products):
wherein
n is an integer from 1 to 18;
R has either α or β stereochemistry;
when n=2 to 18, X has either α or β stereochemistry;
R is OH, O-sugar or O-gallate;
the substituents of C-4, C-6 and C-8 are X, Z and Y, respectively, and bonding of monomeric units occurs at C-4, C-6 or C-8;
when any C-4, C-6 or C-8 are not bonded to another monomeric unit, each X, Y or Z is hydrogen or a sugar; and
the sugar is optionally substituted with a phenolic moiety at any position, for instance, via an ester bond.

The sugar can be selected from the group consisting of glucose, galactose, rhamnose, xylose, and arabinose. The sugar is preferably a monosaccharide or disaccharide. The phenolic moiety is selected from the group consisting of caffeic, cinnamic, coumaric, ferulic, gallic, hydroxybenzoic and sinapic acids. This disclosure is applicable to any formula Aₙ described herein.

When n is 2 and above (e.g. 2 to 18), monomeric units of any formula Aₙ described herein may be bonded via 4→6 and 4→8 linkages. Examples of the compounds of any formula Aₙ described herein are those having the integer n equal 2 to 18; 3 to 18; 2 to 12; 3 to 12; 2 to 5; 3 to 5; 4 to 12; 5 to 12; 4 to 10; or 5 to 10. Thus, procyanidins within the scope of the above formula may be dimers, trimers, tetramers, pentamers, hexamers, heptamers, octamers, nonamers, and decamers. In some embodiments n equals 2, *i.e.,* the compound of formula Aₙ is a dimer. This disclosure is applicable to any formula Aₙ described herein.

In certain embodiments, at least one monomeric unit of any compound of formula Aₙ described herein is an epimer of (-)-epicatechin, (+)-catechin, or of their derivative and has the following formula: 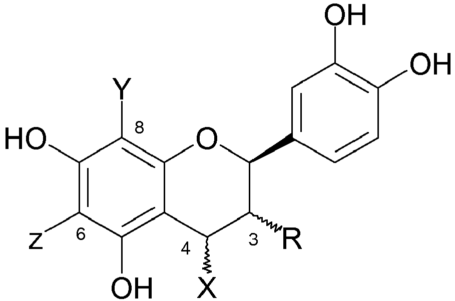 Substituents R, X, Y and Z are as described above. Thus, for example, procyanidins (n is 2 and above) comprising at least one (+)-epicatechin and/or at least one (-)-catechin and/or their derivative (e.g. gallated derivative) are within the scope of the invention.

When n is one (1) in any of the compound of formula Aₙ described herein, the compound of the invention may be (-)-epicatechin, (+)-catechin, or their epimers.

As is known in the art, epimers are diastereoisomers that have the opposite configuration at only one of two or more tetrahedral stereogenic centers, for instance at one of two or more asymmetric carbon atoms. With respect to the type of compounds described herein, an epimer has inverted stereochemical configuration at one of the asymmetric carbon centers C-2 and C-3.

As used herein, the term epimer applies to a compound having an inversion at the C-2 ring carbon atom such that the stereochemical configuration at the C-2 carbon atom is beta. Naturally occurring flavanols and procyanidins typically have alpha stereochemistry at the C-2 carbon atom.

Examples of derivatives of any compound of formula Aₙ described herein include esters, oxidation products, and glucouronidated products. Oxidation products may be prepared, for example, as disclosed in U.S. Pat. No. 5,554,645, the relevant portions of which are incorporated herein by reference. Esters, for example esters with gallic acid, may be prepared using known esterification reactions. Glucuronidated products may be prepared as described in Yu et al, "A novel and effective procedure for the preparation of glucuronides" Organic Letters, 2(16) (2000) 2539-41 and Spencer et al, "Contrasting influences of glucuronidation and O-methylation of epicatechin on hydrogen peroxide-induced cell death in neurons and fibroblasts." Free Radical Biology and Medicine 31(9) (2001) 1139-46.

In one embodiment, the composition comprises an effective amount of the compound having the formula Aₙ, or a pharmaceutically acceptable salt or derivative thereof (including oxidation products): 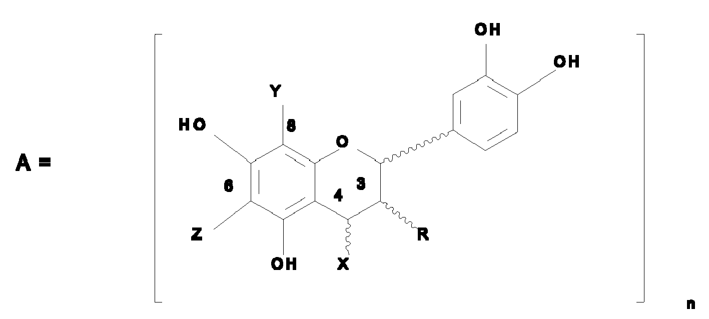 wherein
n is an integer from 1 to 18;
R has either α or β stereochemistry;
when n=2 to 18, X has either α or β stereochemistry;
R is OH;
the substituents of C-4, C-6 and C-8 are X, Z and Y, respectively, and bonding of monomeric units occurs at C-4, C-6 and C-8; and
when any C-4, C-6 or C-8 are not bonded to another monomeric unit, X, Y and Z are hydrogen.

In another embodiment, the invention encompasses the polymeric compound Aₙ having the following formula: 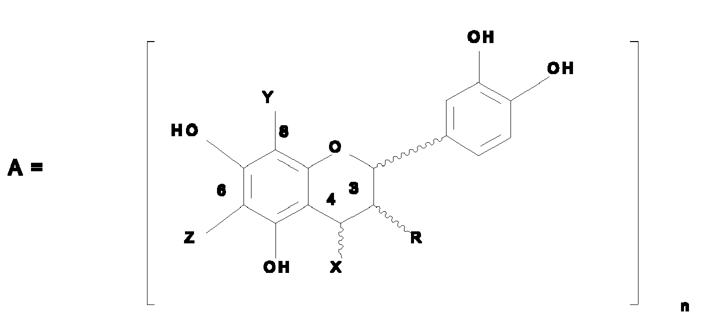 wherein
n is 2;
R and X each have either α or β stereochemistry;
R is OH, O-sugar or O-gallate;
the substituents of C-4, C-6 and C-8 are X, Z and Y, respectively, and bonding of monomeric units occurs at C-4, C-6 and C-8; and
when any C-4, C-6 or C-8 are not bonded to another monomeric unit, each X, Y or Z is hydrogen or sugar;
the sugar is optionally substituted with a phenolic moiety at any position, for instance, via an ester bond.
or a pharmaceutically acceptable salt or derivative thereof (including oxidation products).

In yet another embodiment, the invention encompasses the polymeric compound Aₙ having the following formula: 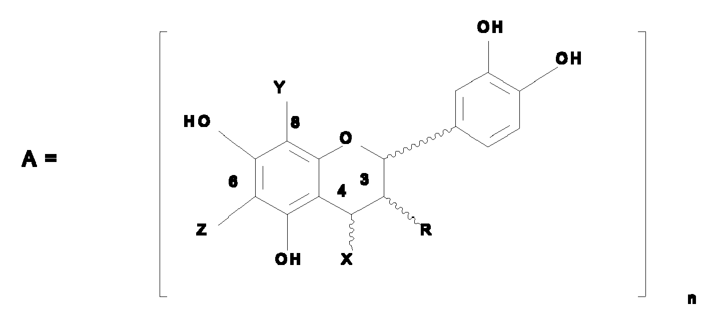 wherein
n is 2;
R and X each have either α or β stereochemistry;
R is OH;
the substituents of C-4, C-6 and C-8 are X, Z and Y, respectively, and bonding of monomeric units occurs at C-4, C-6 and C-8; and
when any C-4, C-6 or C-8 are not bonded to another monomeric unit, X, Y and Z are hydrogen;
or a pharmaceutically acceptable salt or derivative thereof (including oxidation products).

Both purified compounds and mixtures thereof may be used. The degree of purity may be, for example, at least about 50%, or at least about 60%, or at least about 70%, or at least about 80%, or at least about 90%, or at least about 92%, or at least about 95%, or at least about 98%, or at least about 99%. The above degrees of purities may be utilized for any compound of the formula Aₙ, its salts and derivatives.

### Methods of Use

The invention relates to methods for the treatment and prevention of diseases and/or disorders associated with vasoconstriction of peripheral blood vessels and/or small blood vessels (e.g. blood vessels located in arms and legs, fingers and toes, small arteries and arterioles). Any compound and/or composition described in the application may be used to practice the methods described herein.

In certain embodiments the invention provides a method of inducing vasodilation of peripheral blood vessels comprising administering to a human or veterinary animal in need thereof an effective amount of a compound having the formula Aₙ, or a pharmaceutically acceptable salt or derivative thereof (including oxidation products): 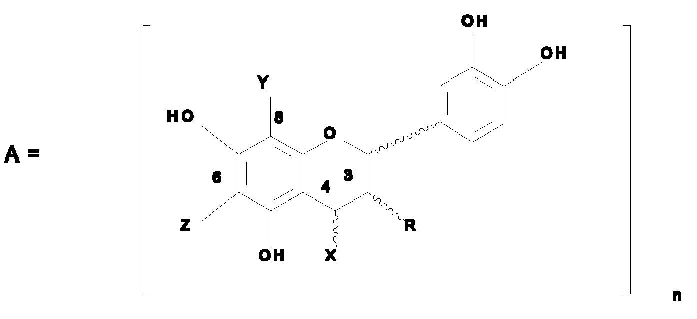 wherein
n is an integer from 1 to 18;
R has either α or β stereochemistry;
when n=2 to 18, X has either α or β stereochemistry;
R is OH, O-sugar or O-gallate;
the substituents of C-4, C-6 and C-8 are X, Z and Y, respectively, and bonding of monomeric units occurs at C-4, C-6 or C-8;
when any C-4, C-6 or C-8 is not bonded to another monomeric unit, each X, Y or Z is hydrogen or a sugar; and
the sugar is optionally substituted with a phenolic moiety at any position, for instance, via an ester bond.

The term "inducing vasodilation of peripheral blood vessels" is used to describe the widening of peripheral blood vessels resulting from relaxation of the muscular wall of the vessels such as to enhance the peripheral blood flow.

Any compound and/or composition described in the application may be used. For example, the above method may involve use of a compound Aₙ, or a pharmaceutically acceptable salt or derivative thereof (including oxidation products), wherein R is OH, and when any C-4, C-6 or C-8 is not bonded to another monomeric unit, X, Y and Z are hydrogen. In some example, a procyanidin dimer or its derivative is administered. In another example, a flavanol may be administered, e.g. (+)-catechin, (-)-epicatechin and their epimers, (+)-epicatechin and (-)-catechin.

Examples of subjects (e.g. a human or a veterinary animal) in need of inducing vasodilation of peripheral blood vessels are those suffering from, or at risk of suffering from, diminished blood flow in such blood vessels. For example, a subject may suffer and/or be at risk of, peripheral vascular disease, e.g. Raynaud's disease, peripheral artery disease (PAD), intermittent claudication (found in subjects suffering from early stages of PAD, this condition results from decreased blood flow to the legs during periods of exercise, including walking/moving around, and causing pain, fatigue or other discomfort in the affected muscle; the discomfort dissipates with the cessation of the activity), vasculitis of small blood vessels, vasospasm, venous thrombosis, venous insufficiency, lymphatic disorders (e.g. lymphatic insufficiency), critical limb ischemia (severe obstruction of the arteries which decreases blood flow to the hands, feet, and legs; one of the symptoms of PAD), acute limb ischemia (an arterial occlusion which suddenly limits blood flow to the arm or leg), atheroembolism (an embolism of lipid debris from an ulcerated atheromatous deposit), and/or lower extremity ischemia (an occlusive disease in arteries supplying blood to lower extremities causing inadequate blood flow).

Thus, the invention provides a method of treating or preventing peripheral vascular disease comprising administering to a human or veterinary animal in need thereof an effective amount of a compound having the formula Aₙ, or a pharmaceutically acceptable salt or derivative thereof (including oxidation products): 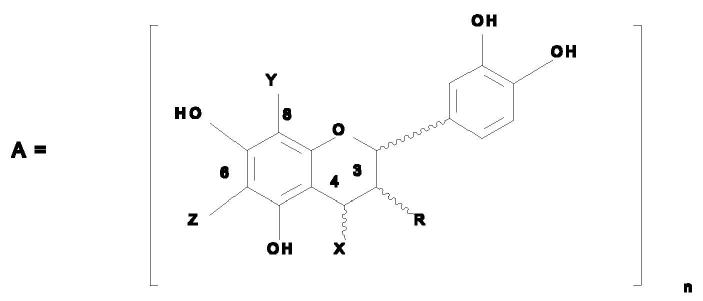 wherein
n is an integer from 1 to 18;
R has either α or β stereochemistry;
when n=2 to 18, X has either α or β stereochemistry;
R is OH, O-sugar or O-gallate;
the substituents of C-4, C-6 and C-8 are X, Z and Y, respectively, and bonding of monomeric units occurs at C-4, C-6 or C-8;
when any C-4, C-6 or C-8 is not bonded to another monomeric unit, each X, Y or Z is hydrogen or a sugar; and
the sugar is optionally substituted with a phenolic moiety at any position, for instance, via an ester bond.

A method of treating or preventing a condition or conditions selected from the group consisting of Raynaud's disease, peripheral artery disease (PAD), intermittent claudication, vasculitis of small blood vessels, vasospasm, venous thrombosis, venous insufficiency, lymphatic disorders (e.g. lymphatic insufficiency), critical limb ischemia, acute limb ischemia, atheroembolism, and lower extremity ischemia is within the scope of the invention.

Any compound described herein may be used to practice the methods of the invention. For example, the above methods may involve use of a compound Aₙ, or a pharmaceutically acceptable salt or derivative thereof (including oxidation products), wherein R is OH, and when any C-4, C-6 or C-8 is not bonded to another monomeric unit, X, Y and Z are hydrogen. In some example, a procyanidin dimer or its derivative is administered. In another example, a flavanol may be administered, e.g. (+)-catechin, (-)-epicatechin and their epimers, (+)-epicatechin and (-)-catechin.

As used herein, "treatment" means improving an existing medical condition, for example, by slowing down the disease progression, prolonging survival, reducing the risk of death, and/or inducing a measurable increase in vasodilation.

The term "preventing" means reducing the risks associated with developing a disease, including reducing the onset of the disease. For example, subjects having a family medical history of conditions recited herein may be suitable for prophylactic treatment.

The above-described methods may be used for treatment/prophylaxis of a veterinary animal, such as a dog, a cat, and a horse.

Thus, the following uses are within the scope of the invention. Use of the compound An, or a pharmaceutically acceptable salt or derivative thereof (including oxidation products), as defined above, in the manufacture of a medicament, food, nutraceutical or dietary supplement for inducing vasodilation of peripheral blood vessels, treating or preventing peripheral vascular disease or any condition selected from the group of: Raynaud's disease, peripheral artery disease (PAD), intermittent claudication, vasculitis of small blood vessels, vasospasm, venous thrombosis, venous insufficiency, lymphatic disorders (e.g. lymphatic insufficiency), critical limb ischemia, acute limb ischemia, atheroembolism, and/or lower extremity ischemia.

The effective amount may be determined by a person of skill in the art using the guidance provided herein and general knowledge in the art. For example, the effective amount may be such as to achieve a physiologically relevant concentration in the body of a mammal. Such a physiologically relevant concentration may be at least 20 nanomolar (nM), preferably at least about 100 nM, and more preferably at least about 500 nM. In one embodiment, at least about one micromole in the blood of the mammal, such as a human, is achieved. The compounds of formula Aₙ, as defined herein, may be administered at from about 50 mg/day to about 1000 mg/day, preferably from about 100-150 mg/day to about 900 mg/day, and most preferably from about 300 mg/day to about 500 mg/day. However, amounts higher than stated above may be used. Flavanol/procyanidin amounts may be determined as described by Adamson, G. E. et al., "HPLC Method for the Quantification of Procyanidins in Cocoa and Chocolate Samples and Correlation to Total Antioxidant Capacity", J. Ag. Food Chem.; 1999; 47 (10) 4184-4188.

The compounds of the invention may be administered acutely or administration continued as a regimen, i.e., for an effective period of time, e.g., daily, monthly, bimonthly, biannually, annually, or in some other regimen, as determined by the skilled medical practitioner for such time as it is necessary. Preferably, the composition is administered daily, most preferably two or three times a day, for example, morning and evening to maintain the levels of the effective compounds in the body of the mammal. To obtain the most beneficial results, the composition may be administered for at least about 30, or at least about 60 days. These regiments may be repeated periodically.

Any of the above methods may be practiced using the compounds of the invention and at least one additional therapeutic agent. Such therapeutic agents may include non-procyanidin therapies that act via the NO-pathway, as well as any other therapeutics, especially those that induce vasodilation or treat vascular diseases. Examples of such agents are lipid lowering therapies, anti-platelet drugs, and blood clotting inhibitors such as heparin.

### Compositions and Formulations

The inventive compounds may be from different sources, of natural origin *(e.g.* genus *Theobroma,* genus *Herrania*) or synthetically prepared. In certain embodiments the compounds are derived from cocoa, including cocoa flavanols and/or cocoa procyanidin oligomers. In one embodiment, these compounds may be extracted from cocoa beans or cocoa ingredients. The term "cocoa ingredient" refers to cocoa solids-containing material derived from shell-free cocoa nibs such as chocolate liquor and partially or fully-defatted cocoa solids (e.g. cake or powder). In addition to, or in place of, the cocoa polyphenols, compositions may contain polyphenols from sources other than cocoa, which have structures and/or properties same or similar to those of cocoa polyphenols. For example, the cocoa polyphenol may be prepared as is known in the art, see e.g. US Pat Nos. 5,554,645; 6,297,273; 6,420,572; 6,156,912; 6,476,241; and 6,864,377, the disclosures of which are hereby incorporated herein by reference.

The compounds of the invention may be prepared by thermally treating (in an aqueous solution) flavanols, procyanidins or their derivatives having alpha stereochemistry at the C-2 atom to cause rotation about the C2 atom resulting in beta stereochemistry at the C-2 atom. This approach is particularly suitable for preparing flavanol epimers, for example, epimers of (-)-epicatechin and (+)-catechin.

Preparation of flavanol epimers may be conducted according to and as described in Freudenberg, K. and Purrmann, L. (1924), Raumisomere Catechin IV. Liebig's Annalen, 437, 472-85; and Fredenberg, K., Bohme, L. and Purrmann, L. (1922), Raumisomere Catechin II. Ber. Dscht. Chem. Ges., 55, 1734-47, the disclosures of which are hereby incorporated herein by reference.

Epimers of procyanidins may be prepared according to the methods described in US Pat Nos. 6,420,572; 6,156,912; 6,476,241; and 6,864,377; and International Appl. Publication WO04/030440 (the disclosures of which are hereby incorporated herein by reference) using flavanol epimers as starting building blocks.

The composition of the invention is useful as a pharmaceutical, a food, a food additive, or a dietary supplement. The compositions may contain a carrier, a diluent, or an excipient. Depending on the intended use, the carrier, diluent, or excipient may be chosen to be suitable for human or veterinary use, food, additive, supplement or pharmaceutical use. The composition may optionally contain an additional vasodilating and/or other therapeutic agent suitable for treatment or prevention of conditions described herein.

As used herein a "food" is a material containing protein, carbohydrate and/or fat, which is used in the body of an organism to sustain growth, repair and vital processes, and to furnish energy. Foods may also contain supplementary substances such as minerals, vitamins and condiments. See Merriam-Webster's Collegiate Dictionary, 10th Edition, 1993. The term food includes a beverage adapted for human or animal consumption. As used herein a "food additive" is as defined by the FDA in 21 C.F.R. 170.3(e)(1) and includes direct and indirect additives. As used herein, a "pharmaceutical" is a medicinal drug. See Merriam-Webster's Collegiate Dictionary, 10th Edition, 1993. A pharmaceutical may also be referred to as a medicament. As used herein, a "dietary supplement" is a product (other than tobacco) that is intended to supplement the diet that bears or contains the one or more of the following dietary ingredients: a vitamin, a mineral, an herb or other botanical, an amino acid, a dietary substance for use by man to supplement the diet by increasing the total daily intake, or a concentrate, metabolite, constituent, extract or combination of these ingredients.

The foods comprising cocoa polyphenols, or any of the compounds described herein, and optionally another therapeutic agent may be adapted for human or veterinary use, and include pet foods. The food may be other than a confectionery, for example a beverage (an example of such a drink and its method of preparation are described in Example 1). Confectionery such as a standard of identity (SOI) and non-SOI chocolate, such as milk, sweet and semi-sweet chocolate including dark chocolate, low fat chocolate and a candy which may be a chocolate covered candy may also be used. Other examples include a baked product (e.g. brownie, baked snack, cookie, biscuit) a condiment, a granola bar, a toffee chew, a meal replacement bar, a spread, a syrup, a powder beverage mix, a cocoa or a chocolate flavored beverage, a pudding, a rice cake, a rice mix, a savory sauce and the like.

A daily effective amount of the compounds of the invention may be provided in a single serving. Thus, a confectionery (e.g. chocolate) or a beverage (e.g. cocoa flavored beverage) may contain at least about 100 mg/serving (e.g. 150-200, 200-400 mg/serving) of the compounds of invention.

Pharmaceuticals containing the inventive compounds, optionally in combination with another therapeutic agents, may be administered in a variety of ways such as orally, sublingually, bucally, nasally, rectally, intravenously, parenterally and topically. A person of skill in the art will be able to determine a suitable mode of administration to maximize the delivery of the compound of formula Aₙ, and optionally another agent. Thus, dosage forms adapted for each type of administration are within the scope of the invention and include solid, liquid and semi-solid dosage forms, such as tablets, capsules, gelatin capsules (gelcaps), bulk or unit dose powders or granules, emulsions, suspensions, pastes, creams, gels, foams or jellies. Sustained-release dosage forms are also within the scope of the invention. Suitable pharmaceutically acceptable carriers, diluents, or excipients are generally known in the art and can be determined readily by a person skilled in the art. The tablet, for example, may comprise an effective amount of the polyphenol-containing composition and optionally a carrier, such as sorbitol, lactose, cellulose, or dicalcium phosphate.

The dietary supplement containing the compounds of the invention, and optionally another therapeutic agent, may be prepared using methods known in the art and may comprise, for example, nutrient such as dicalcium phosphate, magnesium stearate, calcium nitrate, vitamins, and minerals.

Further within the scope of the invention is an article of manufacture such as a packaged product comprising the composition of the invention (e.g. a food, a dietary supplement, a pharmaceutical) and a label indicating the presence of, or an enhanced content of the inventive compounds or directing use of the composition to induce vasodilation of peripheral blood vessels, treat or prevent peripheral vascular disease or any conditions selected from the group of Raynaud's disease, peripheral artery disease (PAD), intermittent claudication, vasculitis of small blood vessels, vasospasm, venous thrombosis, venous insufficiency, lymphatic disorders (e.g. lymphatic insufficiency), critical limb ischemia, acute limb ischemia, atheroembolism, and/or lower extremity ischemia. The packaged product may contain the composition and the instructions for use. The label and/or instructions for use may refer to any of the methods of use described in this application. The invention also relates to methods of manufacturing the article of manufacture comprising any of the compositions described herein, packaging the composition to obtain an article of manufacture and instructing, directing or promoting the use of the composition/article of manufacture for any of the uses described herein. Such instructing, directing or promoting includes advertising.

Also within the scope of the invention is an article of manufacture (such as a packaged product or kit) adapted for use for combination therapy comprising (i) at least one container and at least one compound of the invention (e.g. compound of formula Aₙ), and (ii) at least one additional therapeutic agent *(i.e.,* other than the compound of formula Aₙ), or a pharmaceutically acceptable salt or derivative thereof (including oxidation products), which therapeutic agent may be provided as a separate composition, in a separate container, or in admixture with the compound of the invention.

The invention is further described in the following non-limiting examples.

### EXAMPLES

### Example 1

Peripheral arterial tonometry (PAT) measurements were taken of three volunteers according to the following study. This is a non-invasive technique for examining peripheral microvascular endothelial function. Microvascular endothelial function is assessed by the direct measurement of changes in digital (finger) pulse volume following reactive hyperemia.

Six volunteers were chosen from a group prescreened by blood draw and a health questionnaire. Measurements of height, weight, resting heart rate, and two readings of blood pressure were taken. Volunteers with an average resting blood pressure greater than 160/90 were omitted. Blood samples were analyzed for information regarding complete blood count (CBC), glucose level, general liver function, and lipid panel.

During the study, volunteers were "free living." Twenty four hours prior to each study day, subjects were asked to follow simple dietary guidelines (e.g. to exclude the intake of tea, fruit, cocoa products, wine, etc). In addition, the volunteers were asked to fast overnight for 12 hours prior to the beginning of the study (with water ad libitum). Subjects were not asked to alter their diets on any other days during the investigation period.

On study days, the subjects reported in a fasted state between 7:30 and 9:00 a.m. Body weight, resting heart rate, and blood pressure were all re-measured. The subjects that had an average resting blood pressure of greater than 160/90 were excluded from the study. Subjects then lied down on their backs to acclimatize to ambient conditions. After 30 minutes, a non-invasive, sterile, single-use finger probe was fitted to a finger and a baseline PAT reading was taken and recorded for 10 minutes. The subjects were then subjected to a reactive hyperemia procedure ("RH") which consisted of a period of blood flow occlusion to the measurement arm using an upper arm blood pressure cuff, pressurized to achieve a supra-systolic level (equivalent to about 60mm mercury above systolic blood pressure) for 5 minutes. Then the cuff was released and the data recorded for another five minutes. Following this first reading, subjects drank 3 bottles of Cocoa Drink A (total 483 mg). Over the next six hours, the PAT response was monitored at two, four and six hours.

Cocoa Drink A was prepared as follows: (i) cocoa powder was mixed with water at 80° C for 20 minutes (this step revives any remaining spores in the powder and allows for their destruction during UHT); (ii) the mixture was subjected to UHT treatment at the temperature of 140°C for the period of 6-7 seconds; (iii) the mixture was packaged into an 85ml container and subjected to retort at the maximum water temperature of 115 °C for 10 minutes (total treatment of 19 minutes), and maximum pressure of 2.6 bars. Rotation was applied to help heat transfer. Any variation of the process that can accomplish the functional and/or structural effects described herein can be used.

After a wash-out period (2 or 3 days, see Table 1), 3 subjects (out of six) returned to the lab, had a baseline reading, and were then intravenously infused with L-NMMA. The infusion of L-NMMA occurred over 30 minutes (1mg/kg/min for the first 3 minutes and then 0.2 mg/kg/min for the next 27 minutes). Following infusion of the L-NMMA, 3 bottles of the beverage were fed (483 mg) per subject. Readings were done for baseline, after infusion of L-NMMA and after consumption of cocoa.

The results of the study are represented in Table 1. Referring to Table 1, RH indicates PAT value following a reactive hyperemia procedure; "baseline" refers to baseline reading prior to occlusion period.

| Subject | Subject #1 | | Subject #5 | | *Subject #6* | |
|---|---|---|---|---|---|---|
| | Day 1 | | Day 1 | | *Day 1* | |
| Time | RH | Baseline | RH | Baseline | RH | *Baseline* |
| 0 | 1.77 | 1344 | 1.96 | 1337 | 2.63 | *1557* |
| 2 | 1.89 | 1511 | 2.32 | 1018 | 3.37 | *1364* |
| 4 | 1.72 | 1254 | 2.79 | 604 | 2.52 | *1277* |
| 6 | 1.86 | 1361 | 2.92 | 955 | 2.48 | *1184* |

| Time | Day 9 | | Day 7 | | *Day* 8 | |
|---|---|---|---|---|---|---|
| 0 | 1.93 | 1119 | 1.8 | 1483 | 3.1 | *1159* |
| 2 | 2.31 | 1239 | 2.18 | 694 | 5.03 | *1550* |
| 4 | 2.55 | 991 | 1.69 | 1126 | 3.99 | *1476* |
| 6 | 2.85 | 1088 | 1.88 | 1040 | * | * |

| Time | Day 11 | | Day 10 | | *Day 11* | |
|---|---|---|---|---|---|---|
| *0* | *2.1* | *931* | *2.04* | *1444* | *2.88* | *1374* |
| *LNMMA* | *1.61* | *616* | *1.42* | *689* | *1.75* | *597* |
| *Cocoa* | *2.77* | *689* | *2.46* | *741* | *3.61* | *662* |

The data supports an NO-independent mechanism of action of the compounds described herein. In all three subjects who received LNNMA (an inhibitor of NO synthesis and thus NO-dependent endothelial relaxation), a dramatic reduction in the RH value was observed. When the test drink was then administered, a dramatic increase in the RH value was observed even with LNMMA present in the system. This indicates the blood flow was affected via an NO-independent mechanism. Peripheral blood flow/vasodilation is a function of both NO and NO-independent vasodilators. The endothelium derived NO is one of the most important mediators of vascular tone; however, NO is not the only compound regulating vascular tone. As such, being able to operate via NO-independent mechanisms means that the compound of the invention can exert important vasodilatory effects on the microvasculature which are independent of an intact endothelium. If the endothelium is compromised in any way and simply cannot produce enough NO, the fact that the compounds of the invention act in an endothelium independent fashion offers greater therapeutic benefits, which was previously unexpected.

Further general aspects of the invention are as follows:
1. A method of preventing or treating a peripheral vascular disease comprising administering to a human or a veterinary animal in need thereof an effective amount of a compound having the formula Aₙ, or a pharmaceutically acceptable salt or derivative thereof (including oxidation products): 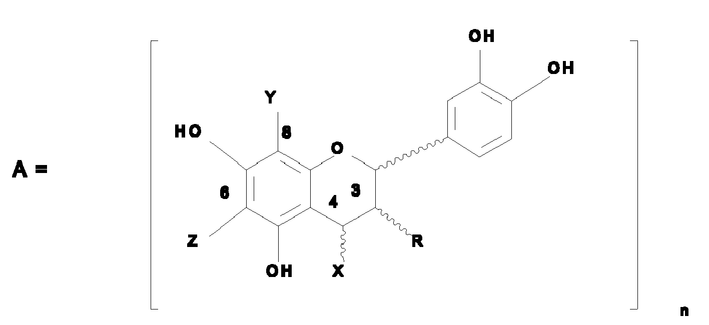 wherein
   n is an integer from 1 to 18;
   R has either α or β stereochemistry;
   when n=2 to 18, X has either α or β stereochemistry;
   R is OH, O-sugar or O-gallate;
   the substituents of C-4, C-6 and C-8 are X, Z and Y, respectively, and bonding of monomeric units occurs at C-4, C-6 or C-8;
   when any C-4, C-6 or C-8 is not bonded to another monomeric unit, each X, Y or Z is hydrogen or a sugar; and
   the sugar is optionally substituted with a phenolic moiety.
2. The method of aspect 1, wherein the peripheral vascular disease is selected from the group consisting of: Raynaud's disease, peripheral artery disease (PAD), intermittent claudication, vasculitis of small blood vessels, vasospasm, venous thrombosis, venous insufficiency, lymphatic insufficiency, critical limb ischemia, acute limb ischemia, atheroembolism, and lower extremity ischemia.
3. The method of aspect 1, wherein the subject is a human suffering from a peripheral vascular disease.
4. The method of aspect 1, wherein the subject is a human suffering from a peripheral artery disease.
5. The method of aspect 1, wherein the subject is a human suffering from intermittent claudication.
6. The method of aspect 1, wherein the subject is a human suffering from Raynaud's disease.
7. The method of aspect 1, wherein R is OH, and when any C-4, C-6 or C-8 is not bonded to another monomeric unit, X, Y and Z are hydrogen.
8. The method of aspect 7, wherein the peripheral vascular disease is selected from the group consisting of: Raynaud's disease, peripheral artery disease (PAD), intermittent claudication, vasculitis of small blood vessels, vasospasm, venous thrombosis, venous insufficiency, lymphatic insufficiency, critical limb ischemia, acute limb ischemia, atheroembolism, and lower extremity ischemia.
9. The method of aspect 7, wherein the subject is a human suffering from a peripheral vascular disease.
10. The method of aspect 7, wherein the subject is a human suffering from a peripheral artery disease.
11. The method of aspect 7, wherein the subject is a human suffering from intermittent claudication.
12. The method of aspect7, wherein the subject is a human suffering from Raynaud's disease.
13. The method of aspect 1, wherein n is 2.
14. A method of treating or preventing a condition selected from the group consisting of Raynaud's disease, peripheral artery disease (PAD), intermittent claudication, vasculitis of small blood vessels, vasospasm, venous thrombosis, venous insufficiency, lymphatic insufficiency, critical limb ischemia, acute limb ischemia, atheroembolism, and lower extremity ischemia comprising administering to a human in need thereof an effective amount of a compound having the formula Aₙ, or a pharmaceutically acceptable salt or derivative thereof (including oxidation products): 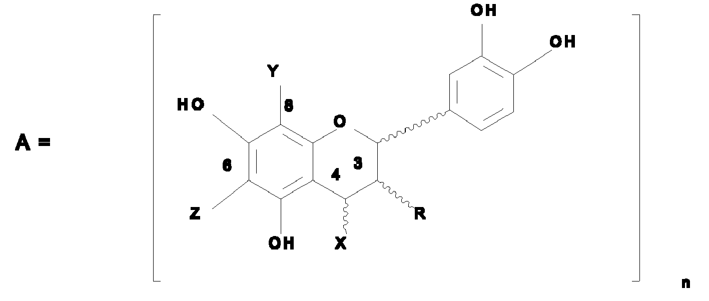 wherein
   n is an integer from 2 to 18;
   R has either α or β stereochemistry;
   X has either α or β stereochemistry;
   R is OH, O-sugar or O-gallate;
   the substituents of C-4, C-6 and C-8 are X, Z and Y, respectively, and bonding of monomeric units occurs at C-4, C-6 or C-8;
   when any C-4, C-6 or C-8 is not bonded to another monomeric unit, each X, Y or Z is hydrogen or a sugar; and
   the sugar is optionally substituted with a phenolic moiety.
15. The method of aspect 1, wherein the compound is epicatechin or a pharmaceutically acceptable salt thereof.
16. The method of aspect 15, wherein the compound is (-)-epicatechin.
17. The method of aspect 1, wherein the compound is provided as a cocoa extract.
18. The method of aspect 1, wherein the compound is provided as a cocoa solid.
19. The method of aspect 18, wherein the cocoa solid is a cocoa powder.
20. An article of manufacture comprising
   (i) a container;
   (ii) a composition within the container, wherein the composition comprises an effective amount of a compound having the formula Aₙ, or a pharmaceutically acceptable salt or derivative thereof (including oxidation products): 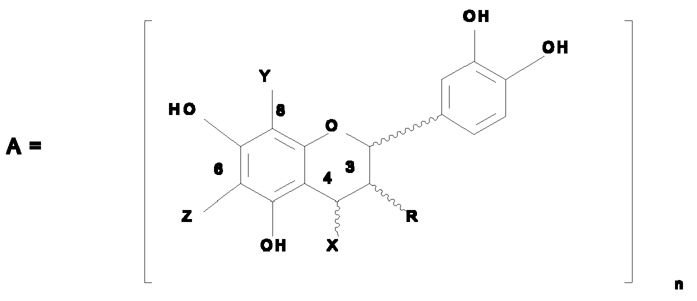 wherein
      n is an integer from 1 to 18;
      R has either α or β stereochemistry;
      when n=2 to 18, X has either α or β stereochemistry;
      R is OH, O-sugar or O-gallate;
      the substituents of C-4, C-6 and C-8 are X, Z and Y, respectively, and bonding of monomeric units occurs at C-4, C-6 or C-8;
      when any C-4, C-6 or C-8 is not bonded to another monomeric unit, each X, Y or Z is hydrogen or a sugar; and
      the sugar is optionally substituted with a phenolic moiety, and
   (iii) instructions directing use of the composition for prevention or treatment of peripheral vascular disease.
21. The article of manufacture of aspect 20, wherein the compound is epicatechin or a pharmaceutically acceptable salt thereof.
22. The article of aspect 21, wherein the compound is (-)-epicatechin.
23. The article of aspect 20, wherein n is 2-18, wherein R is OH, and when any C-4, C-6 or C-8 is not bonded to another monomeric unit, X, Y and Z are hydrogen.

## Claims

1. A composition for use in improving vasodilation in peripheral and/or small blood vessels, treating a condition or disease associated with peripheral vascular circulation and/or small blood cells, or preventing or treating a peripheral vascular disease, comprising a compound having the formula Aₙ, or a pharmaceutically acceptable salt or derivative thereof (including oxidation products): 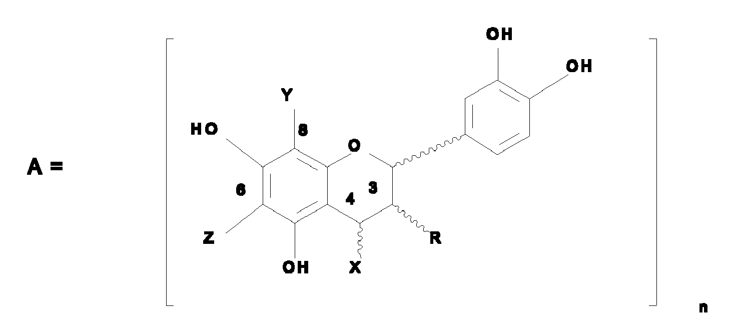 wherein
n is an integer from 1 to 18;
R has either α or β stereochemistry;
when n=2 to 18, X has either α or β stereochemistry;
R is OH, O-sugar or O-gallate;
the substituents of C-4, C-6 and C-8 are X, Z and Y, respectively, and bonding of monomeric units occurs at C-4, C-6 or C-8;
when any C-4, C-6 or C-8 is not bonded to another monomeric unit, each X, Y or Z is hydrogen or a sugar; and
the sugar is optionally substituted with a phenolic moiety.

2. A composition for use according to claim 1, wherein R is OH, and when any C-4, C-6 or C-8 is not bonded to another monomeric unit, X, Y and Z are hydrogen.

3. A composition for use according to claim 1, wherein n is 2.

4. A composition for use according to claim 1, wherein the compound is epicatechin or a pharmaceutically acceptable salt thereof.

5. A composition for use according to claim 4, wherein the compound is (-)-epicatechin.

6. A composition for use according to claim 1, wherein the compound is provided as a cocoa extract.

7. A composition for use according to claim 1, wherein the compound is provided as a cocoa solid.

8. A composition for use according to claim 7, wherein the cocoa solid is a cocoa powder.

9. An article of manufacturing comprising
(i) a container;
(ii) a composition within the container, wherein the composition comprises an effective amount of a compound having the formula Aₙ, or a pharmaceutically acceptable salt or derivative thereof (including oxidation products): 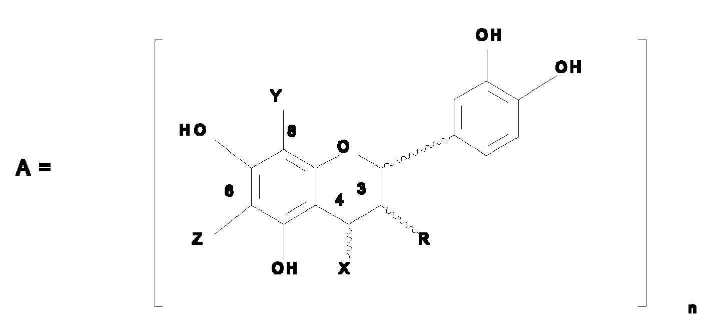 wherein
n is an integer from 1 to 18;
R has either α or β stereochemistry;
when n=2 to 18, X has either α or β stereochemistry;
R is OH, O-sugar or O-gallate;
the substituents of C-4, C-6 and C-8 are X, Z and Y, respectively, and bonding of monomeric units occurs at C-4, C-6 or C-8;
when any C-4, C-6 or C-8 is not bonded to another monomeric unit, each X, Y or Z is hydrogen or a sugar; and
the sugar is optionally substituted with a phenolic moiety, and
(iii) instructions directing use of the composition for improving vasodilation in peripheral and/or small blood vessels, treating a condition or disease associated with peripheral vascular circulation and/or small blood cells, or preventing or treating peripheral vascular disease.

10. An article of manufacture according to claim 9, wherein the compound is epicatechin or a pharmaceutically acceptable salt thereof.

11. An article according to claim 10, wherein the compound is (-)-epicatechin.

12. An article according to claim 9, wherein n is 2-18, wherein R is OH, and when any C-4, C-6 or C-8 is not bonded to another monomeric unit, X, Y and Z are hydrogen.
